# EUROPÄISCHE PATENTSCHRIFT

(11) **EP 0 492 347 B1**
(45) Veröffentlichungstag und Bekanntmachung des Hinweises auf die Patenterteilung: **12.03.1997**
(21) Anmeldenummer: 91121539.0
(22) Anmeldetag: 16.12.1991
(51) Int. Cl.: C07D 307/60

(54) **Verfahren zur Herstellung von threo-4-Alkoxy-5-(arylhydroxymethyl)-2(5H)-furanonen**
Process of preparation of threo-4-alkoxy-5-(arylhydroxymethyl)-2(5H)-furanones
Procédé de préparation des threo-4-alcoxy-5-(arylhydroxyméthyl)-2(5H)-furannones

(30) Priorität: 20.12.1990 CH 4056/90
(43) Veröffentlichungstag der Anmeldung: 01.07.1992
(73) Patentinhaber: LONZA AG, CH-3945 Gampel/Wallis (CH)
(72) Erfinder: Laffan, David, Dr., Visp (Kanton Wallis) (CH)
(74) Vertreter: Weinhold, Peter, Dr.

(56) Entgegenhaltungen:
- EP-A- 0 247 320
- TETRAHEDRON LETTERS Nr. 18, 1979, GREAT BRITAIN Seiten 1627 - 1630; ANDREW PELTER ET AL.: 'The Synthesis of Piperolide and related compounds'
- FIESER AND FIESER'S 'Reagents for Organic Synthesis,Vol.8' 1980 , JOHN WILEY & SONS , NEW YORK

## Beschreibung

Die Erfindung betrifft ein Verfahren zur Herstellung von threo-4-Alkoxy-5-(arylhydroxymethyl)-2(5H)-furanonen der allgemeinen Formel 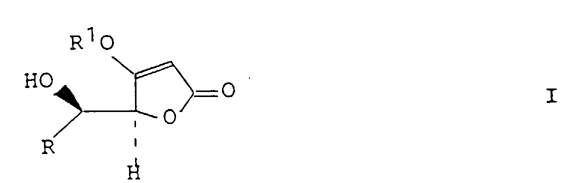 worin R eine gegebenenfalls durch ein oder mehrere Halogenatome und/oder Niederalkylgruppen und/oder eine Nitrogruppe substituierte Phenylgruppe und R¹ eine Alkylgruppe mit 1 bis 4 Kohlenstoffatomen bedeuten.
Die in der Formel I dargestellte Konfiguration ist als relative Konfiguration aufzufassen, sie umfasst auch das Spiegelbild der abgebildeten Formel.

Gegenstand der Erfindung ist insbesondere ein Verfahren zur Herstellung von threo-5-(2-Chlorphenylhydroxymethyl)-4-methoxy-2(5H)-furanon.

threo-4-Alkoxy-5-(arylhydroxymethyl)-2(5H)-furanone sind aus der DE-OS 36 15 157 bekannte pharmazeutische Wirkstoffe mit antikonvulsiver bzw. antiepileptischer Wirksamkeit. Sie wurden bisher in einer mehrstufigen Synthese aus den Enolethern von β-Oxocarbonsäureestern, beispielsweise (E)-3-Methoxy-2-butensäuremethylester, und den entsprechenden Benzaldehyden hergestellt (DE-OS 36 15 157). Diese Synthese liefert zwar stereospezifisch die gewünschte threo-Konfiguration, sie ist jedoch umständlich und die Gesamtausbeute ist gering. Es ist bekannt, dass die 5-Lithio-Derivate von 4-Alkoxy-2(5H)-furanonen (= Tetronsäurealkylestern) mit aromatischen Aldehyden zu 4-Alkoxy-5-(arylhydroxymethyl)-2(5H)-furanonen kondensiert werden können (A.Pelter et al., J.Chem.Soc.Perkin Trans.I, 1987, S.717). Zur Herstellung der 5-Lithiotetronsäureester wurden die Tetronsäureester mit Lithiumdiisopropylamid oder n-Butyllithium unter Schutzgas bei -78°C umgesetzt, was für eine Herstellung in technischem Massstab aus Kostenund Sicherheitsgründen kaum akzeptabel ist. Auch die Kondensation mit den Aldehyden wurde unter diesen Bedingungen durchgeführt. Als Produkte wurden stets Gemische der threo- und erythro-Diastereomere erhalten, deren Trennung oft schwierig ist.

Aufgabe der vorliegenden Erfindung war daher, ein Verfahren zur Herstellung von 4-Alkoxy-5-(arylhydroxymethyl)-2(5H)-furanonen zur Verfügung zu stellen, das möglichst in einer Stufe, ausgehend von kommerziell erhältlichen Materialien, sowie ohne Verwendung von lithiumorganischen Reagenzien in hoher Ausbeute stereospezifisch die threo-Konfiguration liefert. Erfindungsgemäss wird diese Aufgabe durch das Verfahren nach Patentanspruch 1 gelöst.

Es wurde überraschend gefunden, dass durch Verwendung von Lithiumhydroxid als Katalysator 4-Alkoxy-2(5H)-furanone direkt mit Benzaldehyden kondensiert werden können, ohne dass hierfür tiefe Temperaturen, Schutzgas oder wasserfreie Lösungsmittel erforderlich wären.
4-Methoxy-2(5H)-furanon ist neuerdings auch kommerziell erhältlich, ein Herstellungsverfahren ist in der DE-PS 28 45 037 beschrieben. Analog können auch andere 4-Alkoxy-2(5H)-furanone hergestellt werden.

Die Reaktion wird zweckmässig in einer Mischung eines dipolaren aprotischen Lösungsmittels und eines protischen Lösungsmittels durchgeführt, vorzugsweise in Acetonitril/Wasser. Das Volumenverhältnis des aprotischen zu dem protischen Lösungsmittel liegt vorteilhaft zwischen 20:1 und 1:20, vorzugsweise zwischen 10:1 und 1:1.

Als Lithiumhydroxid wird vorzugsweise die billigere Monohydratform eingesetzt, die Verwendung von wasserfreiem Lithiumhydroxid bringt keine zusätzlichen Vorteile.

Die Reaktionstemperatur liegt vorteilhaft zwischen 0 und 60°C, vorzugsweise zwischen 10 und 40°C.

Ebenfalls überraschend wurde gefunden, dass es von Vorteil ist, wenn, entgegen der sonst üblichen Arbeitsweise, das basische Reaktionsgemisch vor der Aufarbeitung nicht neutralisiert wird. Setzt man nämlich lediglich Wasser zu und destilliert das organische Lösungsmittel ganz oder weitgehend ab, so wird als Produkt nur das gewünschte threo-Diastereomer in fester Form isoliert. Anscheinend stehen threo- und erythro-Diastereomere in basischer Lösung im Gleichgewicht und es kristallisiert nur das schwerer lösliche threo-Diastereomer aus. Wird das Reaktionsgemisch dagegen neutralisiert, so kann sich das Konfigurationsgleichgewicht nicht mehr neu einstellen, wenn eine Form auskristallisiert und demzufolge das bei der Reaktion entstandene erythro-Diastereomer sich nicht nachträglich in die threo-Form umwandeln.

Nachdem das Produkt isoliert ist, kann es auf übliche Weise, z.B. durch Umkristallisieren, weiter gereinigt werden.

Die nachfolgenden Beispiele verdeutlichen die Durchführung des erfindungsgemässen Verfahrens.

### Beispiel 1

### (±)-threo-5-(2-Chlorphenylhydroxymethyl)-4-methoxy-2(5H)-furanon

In 200 ml Acetonitril wurden 20,0 g 4-Methoxy-2(5H)-furanon (175 mmol), 24,6 g 2-Chlorbenzaldehyd (175 mmol) und 1,47 g Lithiumhydroxid-monohydrat (35,4 mmol) bei Raumtemperatur unter Rühren gelöst. Nach 30 min wurden 40 ml Wasser zugegeben und nach weiteren 60 min wurde das Acetonitril bei 40°C Badtemperatur im Vakuum abdestilliert.

Die zurückbleibende wässrige Suspension wurde mit 250 ml Wasser verdünnt und 2 h bei Raumtemperatur gerührt. Das ausgefallene Produkt wurde durch Filtration isoliert, bei 35°C und 1 mbar getrocknet und aus 170 ml Ethylacetat umkristallisiert.
- Ausbeute:: 30,7 g (69,8% der Theorie)
- Schmp.:: 149-150°C

| | | | |
|---|---|---|---|
| Analyse: | Ber. | C 56,69 | H 4,35 |
| | Gef. | C 56,8 | H 4,2 |

Die ¹H- und ¹³C-NMR-Spektren und die chromatographischen Eigenschaften des Produktes stimmen mit den Literaturwerten überein.

### Beispiel 2

### (±)-threo-4-Methoxy-5-(phenylhydroxymethyl)-2(5H)-furanon

In 20 ml Acetonitril wurden 2,0 g 4-Methoxy-2(5H)-furanon (17,5 mmol), 1,86 g Benzaldehyd (17,5 mmol) und 148 mg Lithiumhydroxid-monohydrat (3,5 mmol) bei Raumtemperatur unter Rühren gelöst. Nach 30 min wurden 4 ml Wasser zugegeben, und nach weiteren 30 min wurde das Acetonitril bei 40°C Badtemperatur im Vakuum abdestilliert.

Die zurückbleibende wässrige Suspension wurde mit 25 ml Wasser verdünnt und 2 h bei Raumtemperatur gerührt. Das ausgefallene Produkt wurde durch Filtration isoliert, bei 35°C und 1 mbar getrocknet und aus 60 ml Dichlormethan umkristallisiert.

Ausbeute: 2,2 g (58%); Schmp.: 156-157°C

¹H-NMR-Spektrum und Schmelzpunkt stimmen mit den Literaturwerten überein.

## Patentansprüche

1. Verfahren zur Herstellung von threo-4-Alkoxy-5-(arylhydroxymethyl)-2(5H)-furanonen der allgemeinen Formel und relativen Konfiguration 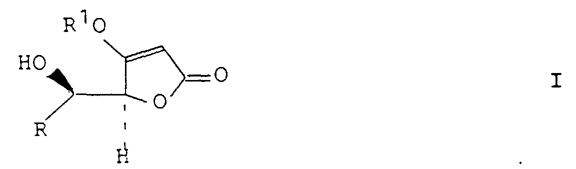 worin R eine gegebenenfalls durch ein oder mehrere Halogenatome und/oder Niederalkylgruppen und/oder eine Nitrogruppe substituierte Phenylgruppe und R¹ eine Alkylgruppe mit 1 bis 4 Kohlenstoffatomen bedeuten, durch Kondensation eines 4-Alkoxy-2(5H)-furanons der allgemeinen Formel 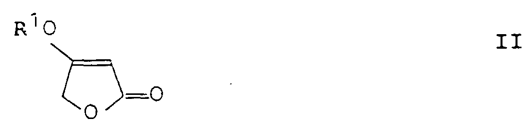 worin R¹ die oben genannte Bedeutung besitzt, mit einem gegebenenfalls substituierten Benzaldehyd der allgemeinen Formel
R - CHO III
worin R die oben genannte Bedeutung besitzt, dadurch gekennzeichnet, dass die Kondensation unter Katalyse durch Lithiumhydroxid in einer Mischung eines dipolaren aprotischen Lösungsmittels mit einem protischen Lösungsmittel im Volumenverhältnis 20:1 bis 1:20 durchgeführt wird und das Produkt durch Wasserzusatz und Entfernen des dipolaren aprotischen Lösungsmittels ohne vorheriges Neutralisieren ausgefällt wird.

2. Verfahren nach Patentanspruch 1, dadurch gekennzeichnet, dass das Volumenverhältnis aprotisches Lösungsmittel:protisches Lösungsmittel 10:1 bis 1:1 beträgt.

3. Verfahren nach Patentanspruch 1 oder 2, dadurch gekennzeichnet, dass als dipolares aprotisches Lösungsmittel Acetonitril und als protisches Lösungsmittel Wasser eingesetzt wird.

4. Verfahren nach Patentanspruch 3, dadurch gekennzeichnet, dass das Acetonitril nach Wasserzusatz durch Destillation unter normalem oder vermindertem Druck entfernt wird.

5. Verfahren nach mindestens einem der Patentansprüche 1 bis 4, dadurch gekennzeichnet, dass die Kondensation bei einer Temperatur von 0 bis 60°C durchgeführt wird.

6. Verfahren nach Patentanspruch 5, dadurch gekennzeichnet, dass die Kondensation bei einer Temperatur von 10 bis 40°C durchgeführt wird.

7. Verfahren nach mindestens einem der Patentansprüche 1 bis 6, dadurch gekennzeichnet, dass R¹ eine Methylgruppe ist.

8. Verfahren nach mindestens einem der Patentansprüche 1 bis 7, dadurch gekennzeichnet, dass R eine 2-Chlorphenyl-Gruppe ist.

## Claims

1. A process for the production of threo-4-alkoxy-5-(arylhydroxymethyl)-2(5H)-furanones of the general formula and relative configuration: 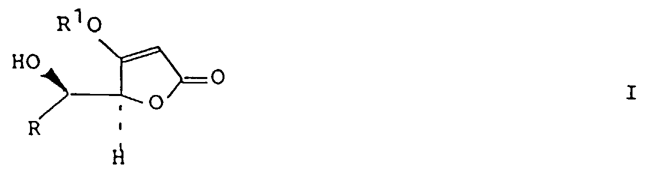 wherein R is a phenyl group optionally substituted by one or more halogen atoms and/or lower alkyl groups and/or a nitro group, and R¹ is an alkyl group with 1 to 4 carbon atoms, by condensation of a 4-alkoxy-2(5H)-furanone of the general formula: 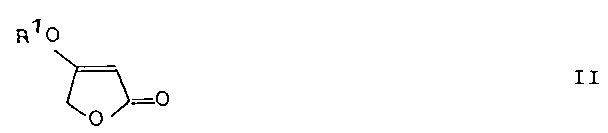 wherein R¹ has the above-mentioned meaning, with an optionally substituted benzaldehyde of the general formula:
R - CHO III
wherein R has the above-mentioned meaning, characterized in that the condensation is performed under catalysis by lithium hydroxide in a mixture of a dipolar aprotic solvent with a protic solvent in the volume ratio of 20:1 to 1:20; and that the product is precipitated by addition of water and removal of the dipolar aprotic solvent without previous neutralization.

2. The process according to claim 1, characterized in that the volume ratio of aprotic solvent:protic solvent is 10:1 to 1:1.

3. The process according to claim 1 or 2, characterized in that acetonitrile is used as the dipolar aprotic solvent and water is used as the protic solvent.

4. The process according to claim 3, characterized in that the acetonitrile is removed after the addition of water by distillation under standard or reduced pressure.

5. The process according to at least one of the claims 1 to 4, characterized in that the condensation is performed at a temperature of 10 to 60 °C.

6. The process according to claim 5, characterized in that the condensation is performed at a temperature of 10 to 40 °C.

7. The process according to at least one of the claims 1 to 6, characterized in that R¹ is a methyl group.

8. The process according to at least one of the claims 1 to 7, characterized in that R is a 2-chlorophenyl group.

## Revendications

1. Procédé pour la préparation de thréo-4-alcoxy-5-(arylhydroxyméthyl)-2(5H)-furanones selon la formule générale et la configuration relative 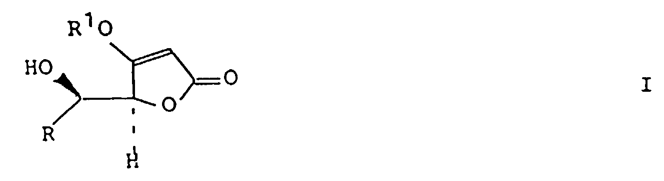 dans laquelle R signifie un groupe phényle éventuellement substitué par un ou plusieurs atomes d'halogène et ou des groupes alkyle inférieur et ou un groupe nitro et R¹ signifie un groupe alkyle avec un jusqu'à quatre atomes de carbone, par condensation d'une 4-alcoxy-2(5H)-furanone de la formule générale 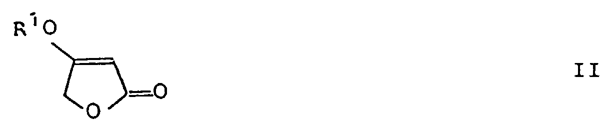 dans laquelle R¹ possède la signification précitée avec un benzaldéhyde éventuellement substitué de la formule générale
R - CHO III
dans laquelle R possède la signification précitée, caractérisé en ce que la condensation s'effectue sous catalyse par hydroxyde de lithium dans un mélange d'un solvant aprotique dipolaire avec un solvant protique dans un rapport volumique 20:1 jusqu'à 1:20 et le produit précipite par addition d'eau et extraction du solvant aprotique dipolaire sans neutralisation préalable.

2. Procédé selon la revendication 1, caractérisé en ce que le rapport volumique solvant aprotique : solvant protique est de 10:1 jusqu'à 1:1.

3. Procédé selon la revendication 1 ou 2, caractérisé en tant que solvant aprotique dipolaire on met en oeuvre de l'acétronitrile et comme solvant protique on utilise de l'eau.

4. Procédé selon la revendication 3, caractérisé en ce que l'on sépare l'acétonitrile après addition d'eau par distillation sous pression normale ou réduite.

5. Procédé selon au moins l'une des revendications 1 à 4, caractérisé en ce que la condensation est conduite à une température de 0 à 60°C.

6. Procédé selon la revendication 5, caractérisé en ce que la condensation s'effectue à une température de 10 à 40°C.

7. Procédé selon au moins l'une des revendications 1 à 6, caractérisé en ce que R¹ est un groupe méthyle.

8. Procédé selon au moins l'une des revendications 1 à 7, caractérisé en ce que R est un groupe 2-chlorophényle
